# EUROPEAN PATENT SPECIFICATION

(11) **EP 3 806 878 B1**
(45) Date of publication and mention of the grant of the patent: **05.06.2024**
(21) Application number: 18792388.3
(22) Date of filing: 17.10.2018
(51) Int. Cl.: A61K 36/11, A61K 36/18, A61K 36/36, A61K 36/33, A61K 36/53, A61K 36/899, A61K 36/28, A61K 36/47, A61P 13/02, A61P 13/04, A61P 13/12, A61P 19/06, A61P 1/06, A61P 31/04, A61P 39/00

(54) **HERBAL COMPOSITIONS FOR THE TREATMENT OF UROLITHIASIS AND URINARY TRACT INFECTIONS**
KRÄUTERZUSAMMENSETZUNGEN ZUR BEHANDLUNG VON UROLITHIASIS UND HARNWEGSINFEKTIONEN
COMPOSITIONS À BASE D'HERBES MÉDICINALES POUR LE TRAITEMENT DE L'UROLITHIASE ET D'INFECTIONS DES VOIES URINAIRES

(30) Priority: 14.06.2018 TR 201808522
(43) Date of publication of application: 21.04.2021
(73) Proprietor: Setonda, S.L., 08390 Montgat Barcelona (ES)
(72) Inventor: YUSUBOV, Natig, ESENLER ISTANBUL (TR)
(74) Representative: Durán-Corretjer, S.L.P.
(86) International application number: PCT/EP2018/078444
(87) International publication number: WO 2019/238254

(56) References cited:
- ES-A1- 2 143 439
- US-A1- 2010 092 586
- US-A1- 2013 064 912
- US-A1- 2016 213 726
- WRIGHT ET AL: "Herbal medicines as diuretics: A review of the scientific evidence", JOURNAL OF ETHNOPHARMACOLOGY, ELSEVIER IRELAND LTD, IE, vol. 114, no. 1, 8 October 2007 (2007-10-08), pages 1-31, XP022266034, ISSN: 0378-8741, DOI: 10.1016/J.JEP.2007.07.023
- GONZALEZ-TEJERO ET AL: "Medicinal plants in the Mediterranean area: Synthesis of the results of the project Rubia", JOURNAL OF ETHNOPHARMACOLOGY, ELSEVIER IRELAND LTD, IE, vol. 116, no. 2, 14 December 2007 (2007-12-14), pages 341-357, XP022486285, ISSN: 0378-8741, DOI: 10.1016/J.JEP.2007.11.045
- GRASES1 F ET AL: "Urolithiasis and Phytotherapy", INTERNATIONAL UROLOGY AND NEPHROL, AKADEMIAI, BUDAPEST, HU, vol. 26, no. 5, 1 September 1994 (1994-09-01), pages 507-511, XP009110535, ISSN: 0301-1623, DOI: 10.1007/BF02767650
- FORTINO SOL?RZANO-SANTOS ET AL: "Essential oils from aromatic herbs as antimicrobial agents", CURRENT OPINION IN BIOTECHNOLOGY, vol. 23, no. 2, 1 April 2012 (2012-04-01), pages 136-141, XP055116275, ISSN: 0958-1669, DOI: 10.1016/j.copbio.2011.08.005
- SR GHATAPANADI* ET AL: "Documentation of folk knowledge on medicinal plants of Gulbarga district, Karnataka", INDIAN JOURNAL OF TRADITIONAL KNOWLEDGE, RESOURCES, NEW DELHI, NEW DELHI - INDIA, vol. 10, no. 2, 1 April 2011 (2011-04-01), pages 349-353, XP018029406, ISSN: 0972-5938

## Description

### Technical Field

This invention relates to herbal and pharmaceutical compositions for use in the treatment of urolithiasis (kidney stones), and prophylaxis of re-formation of stones after passing them, the treatment of gout, and in prevention of excessive amount of salts (urates, oxalates, phosphates, carbonates) in kidneys, the treatment of dysuria phenomenon (pollakiuria, strangury, dysuria, nycturia, low urinary pressure and urinary continence), and the treatment of urinary and biliary tract spasm, the treatment of asymptomatic bacteriuria and the treatment of urinary tract infections (cystitis, urethritis, pyelonephritis etc.). The invention also refers to a production method of said herbal compositions.

### State of the Art

Kidney stone is the name of solid mineral substances accumulating in kidneys, also known as "nephrolithiasis" or "urolithiasis" in medicine. If substances such as calcium oxalate and uric acid present more density than expected in normal condition inside urinary, kidney stones are formed. Those substances can settle in kidney in the form of crystals and form kidney stones by growing in time. The stones can be discharged from body by the transposition of stones or by the movement of stones downward along urinary canals. However, stones preventing urine flow by sticking at any section of urinary canal cause usually feared, severe typical pain in kidney.

Urinary tract infections are the most frequently encountered bacterial infections by the physicians. Nearly 50% of women face urinary tract infection in any period of their lives. Cystitis, which is one of urinary tract infections, is a bladder inflammation reaction. It is encountered usually on women. The patient consults a doctor with dysuria, pollakiuria, urge sensation. Pyelonephritis, which is another urinary tract infection, is a bacterial infection of renal parenchyma. Urethritis is the name of urethra (ejaculatory duct of bladder) inflammation case. In general, it is also called as NSU (non-specific urethritis) or NGU (non-gonococcic urethritis).

Stones formed in kidney can create great pains in patients from time to time. Those pains differ according to its size and particularly large stones need to be treated immediately. Small stones need to be waited for their spontaneously passing. In the state of the art, medication therapy is administered within that period.

When regulating medication, protein binding of drug, effect of drug on metabolism and drug absorption should be taken into consideration. There is plurality of medicines for passing kidney stone but the main factor hereby depends on the level of disease. Many products are ineffective when kidney stone is in a form such that it would damage organs of the patient and it would make the life of patient miserable due to pains. Those kind of medicines assists in stone passing since they cause smooth muscles on bladder outlet to relax. When the smooth muscles relax then easy urination is realized.

Pain killers are additionally given to relieve pains together with those medicines and thereby increasing drug use and side effects.

In the state of the art there are also some herbal methods used for passing kidney stones. Uva ursi is beneficial in the treatment of renal infections as well as being helpful against the stones. It relieves pains and cleans urinary tract. However, use of it is particularly preferred since it may lead to certain problems when daily dose of that herb is exceeded.

Another method is consuming taraxacum root regularly by brewing as tea. It cleanses kidneys and helps for dissolution of kidney stones. Furthermore, it necessitates use of an herb such as basil and eases the passing of kidney stones via urinary tract. The amounts of those herbs used are particularly important. When patients consume those herbs without consulting they cannot determine sufficient effective dose and/or undesired effects can be faced when they exceed recommended daily allowance.

Too much side effects of medicines used in the state of the art, not being able to determine effective dose of herbs by patients has prompted the inventor to formulate novel herbal and pharmaceutical compositions.

One of the most significant herbs used in the treatment of inflammation of kidney, urinary tract and bladder is equisetum arvense which is also called as horsetail. Horsetail family-Equisetaceae family is a perennial plant species growing in arctic and mild regions of north hemisphere. They do not bloom whereas they reproduce by spore. They develop 15-25 cm height sponges in March, April period. It is known to have firming/reinforcing, diuretic, primarily leg oedema dissolving, wound healing, chronic antitussive, lung analeptic, blood cleansing effects. It can be utilized successfully in inflammation cases of kidney, urinary tract and bladder. This herb is known to comprise a plurality of flavonoids, alkaloids, minerals, phenolic petrosins, triterpenoids, saponins, phytosterols. Although it behaves like a slight diuretic, it is used for the treatment of sleep wetting of children and incontinence. As a diuretic, it particularly effective on metabolic or hormonal oedema. It has a high capacity of removing/eliminating water from body and enables 30% more urination than normal. Most of the products comprising equisetum arvense are sold as weight loss supplement. This property is due to effects of several components among which equisetonin and potassium is found. (Mamedova KT, Gysejnova ID. Effect of Equisetum arvense L. On diuresis. Doklady - Akad NaukAzerb 1996; 51: 175-179).

Spergularia rubra known as having diuretic effect is also called as; Sandwort, Red sandspurry, spergularia or red spergularia. Spergularia is a 20-25 cm height plant. Its small five-leafed flowers have pink colors. It is in flower from the ends of spring till the ends of autumn. It grows on sandy hillsides, fields, meadows and grasslands for years. It prefers sandy and clayish soils. Spergularia rubra which is a medical herb is included in content of some medicines. Preparations made of this herb has diuretic, spasm eliminating, anti-inflammatory, anti-bacterial and antioxidant effects. Its use as diuretic, antiseptic for the treatment of diseases concerning renal system is known. (AI-Quran S. Taxonomical and pharmacological survey of therapeutic plants in Jordan. J Nat Prod 2008;1 :10-26.; Gonzalez-Tejero MR, Casares-Porcel M, Sanchez-Rojas CP, Ramiro-Gutierrez JM, Molero-Mesa J, Pieroni A, et al. Medicinal plants in the Mediterranean area: synthesis of the results of the project Rubia.JEthnopharmacol 2008;116:341-57).

Boldo- peumus boldus, namely Boldo is a herb species natively endemic to the cost regions of Chile. It is from Monimiaceae family which is very close to Lauraceae family. Peumus boldus is used as hepatic function regulator due to its rich phenolic and alkaloid content, antispasmodic, digestive stimulant and neuroleptic. It contains essential oils and several alkaloids. Leaves of boldo are used in making tea and tincture, wherein its extract is used in making natural medicine.

Prickly pear- Opuntia Ficus indica is; a typical species of Opuntia genus classified in indian fig, Opuntia ficus indica cactus family. In Turkish, this cactus species is also called as Frankish fig, Frankish fruit, prickly fig. Opuntia ficus indica is used for the treatment of burns, wounds, oedema, hyperlipidemia, obesity and catarrhal gastritis. Alcohol extracts are indicated for anti-inflammatory, hypoglysemic and antiviral purposes. (Kaur, M.; Kaur, A.; Sharma, R. Pharmacological actions of Opuntia ficus indica: A Review. J. Appl. Pharm. Sci. 2012, 2, 1).

Sideritis angustifolia sideritis scardica griseb., Lamiaceae (highland, hill tea) also called as sideritis, hill tea, highland tea, an endemic plant of Balkan Peninsula, is used in conventional medicine for the treatment of antimicrobial infections, gastrointestinal compliments, inflammatory and rheumatic diseases.

Rosmarinus officinalis commonly known as rosemary is a woody, evergreen perennial plant having a smell, and having needle-like, white, pink, purple or blue leaves. This plant belonging to Labiatae (Lamiaceae) family contains in its chemical structure flavonoids and phenolic acids, terpenoids, volatile oils. Among the popular uses of rosemary, uses for digestion, choleretic, emmenagogue, sedative, anti-spasmodic, hypertensive, sudorific, wound healing, antiseptic and analgesic properties and as capillary growth stimulant (in topical administrations) are present. It is used internally as anti-flatulent, digestion system stimulant, bile increasing, sudorific and diuretic. It has antispasmodic effects on bile and small intestine. It is central nervous system stimulant. It has antioxidant activities. It accelerates bloodstream externally, and is used as pain killer for muscle and rheumatic pains and is used for festering wounds. It has hair loss preventer and hair refreshing effect when made tea and applied on hair and made friction. Furthermore, it is used as volatile oil or leaf extracts in natural foot care products. In China, it is recommended for headaches, insomnia and mental fatigue. It is used for its carminative (anti-flatulent) and anti-migraine properties in India. Zeng H. et al. (2001) have searched antioxidant effect of rosemary and its effects mechanism. They have particularly searched bicarbonol, rosmanol and epirosmanol phenolic diterpenes capacity to inhibit lipid peroxidation of low intensity lipoprotein (LDL) in blood and cell membranes specifically as well as anti-radical effect of these diterpenes. Obtained result showed that carnosol, rosmanol and epirosmanol has the capacity to inhibit Cu2 + mediated oxidation of LDL and cell membranes. Moreover, it is determined that this activity depends on the anti-radical activity of these diterpenes. It also has anti-inflammatory effects. It has been found by means of conducted studies that its rosmarinic acid has anti-inflammatory effects on rat models which is carrageenin-induced plantar oedema. (Alonso J., (2004). Al-Sereiti MR. et al (1999)) Specially in cosmetics sector, rosemary-eco extract is often used for sensitive and irritated skins. Studies also proving antimicrobial effect of rosmarinus officinalis plant exist in the state of the art (Moreno S, et al., 2006).

Cynodon dactylon also known as dog's tooth grass is a fast-growing perennial warm temperatures plant. It is in Poaceae family. Bermuda grass which called as dog's tooth grass in Turkey is used commonly in Mediterranean climate belt. Cynodon is bitter, odorous, appetizing, antihelmintic, antifebrile in comparison to Ayurveda of India conventional pharmacopeia. It is used in cases of leukoderma, bronchitis, asthma, tumors and hypersplenism. It is used for the treatment of any kind of bleeding and skin problem in homoeopathic medicine systems. It has volatile oils, glycosides, saponins, tannins, flavonoids and carbohydrates in its chemical composition. As a result of conducted studies, its anti-diabetic activity, anti-arrhythmic activity, anti-inflammatory activity, central nervous system activity, immunomodulator activity, diuretic activity, antioxidant activity, chemotherapeutic activity is known. Cynodon dactylon extract has a beneficial effect in preventing calcium oxalate deposition in kidneys and eliminating them. Such results are a scientific explanation for its use for the treatment of kidney stone. (Atmani F, Sadki C, Aziz M, Mimouni M, Hacht B.,Urol Res. 2009 Apr: Cynodon dactylon extract as a preventive and curative agent in experimentally induced nephrolithiasis).

Melissa officinalis is also known as Lemon balm and balm mint. Melissa officinalis L. is one of the oldest and most popular medical herbs. It is in the Lamiaceae family known for its many aromatic and medicinal herbs commonly used in the field of conventional medicine and gastronomy of Europe. Lemon balm originally native to Mediterranean Region, become widespread in South Slovakia and Moravia flora. (BERTOVÁ, L. - GOLlAŠOVÁ, K. - eds.: Flóra Slovenska, Vol. V/1. Bratislava, Veda 1993, p. 315-316). Most commonly known therapeutic properties of Lemon balm are mitigant, carminative, antispasmodic, antibacterial, anti-viral, anti-inflammatory and anti-oxidative. (WAGNER, H. - SPRINKMEYER, L.: Über die pharmakologische Wirkung von Melissengeist. Dtsch. Apoth. Ztg. 113, 1973, p. 1159-1166). (YAMASAKI, K. - NAKANO, M. - KAWAHATA, T. - MORI, H. - OTAKE, T. - UEBA, N. - OISHI, I. - INAMI, R. - YAMANE, M. - NAKAMURA, M. - MURATA, H. - NAKANISHI, T.: Anti-HIV-1 activity of herbs in Labiatae. Biol. Pharm. Bull. 21, 1998, p. 829-833).

Drug is dried leaves of Melissa officinalis plant and dry drug should contain at least 1% rosmarinic acid ((European Pharmacopoeia, 7th Ed., Vol. 1, Strasbourg: Council of Europe, 1184-5, 2010) Cream containing Melissa extract with externally lyophilized water in the ratio of 1% is used 2-4 times a day from the start of first symptoms till 1-2 days after the lesions are healed for the treatment of Herpes labialis (WHO. WHO Monographs on Selected Medicinal Plants, Vol. 1, World Health Organization, Geneva, 1999; ESCOP Monographs, The Scientific Foundation for Herbal Medicinal Products, 2nd Edition, completely revised and expanded, Thieme, 354-358, 2003). It is used internally as carminative and spasmolytic for gastrointestinal system disorders; also as sedative for stress, discomfort, irritability and sleep problems rather in form of infusion and its tincture, irrespective of time limitation. Infusion is used 2-3 times a day by being prepared from 2-3 g drug; and its tincture prepared with 45% ethanol is used 3 times a day 2-6 ml (WHO. WHO Monographs on Selected Medicinal Plants, Vol. 1, World Health Organization, Geneva, 1999 - Baykan Erel 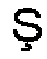 , "Melissa officinalis L." Tedavide Kullamlan Bitkiler FFD Monograflan, Demirezer O, Ersöz T, Saraçoǧlu Ð, 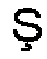 ener B (Eds.), 2. Baski, MN Medical & Nobel Tip Kitabevi, Ankara, 383- 90, 2011) In the work called Materia Medica of Dioscorides, it is stated that decoction of Melissa officinalis (melissophullon) is used against scorpion stings and toothache and also it is recommended for women to use it after having shower (Dioscorides de Materia Medica, Being an Herbal with many other Medicinal Materials written in Greek in the first century of the common era a new indexed version in modern English by TA Osbaldeston and RPA Wood, IBIDIS Press, Johannesburg South Africa, 2000).

ES 2 143 439 A1 discloses a reducer of kidney stones and uric acid comprising Horsetail (Equisetum arvense) 43.5% by weight, b) Boldo (Peumus boldus) 22.0% by weight, c) Prickly pear (Opuntia ficus-indica) 11.5% by weight, d) Melissa (Melissa officinalis) 11.5% by weight, and e) Rosemary (Rosmarinus officinalis).

### Description of the Invention

The present invention relates to herbal and pharmaceutical compositions comprising Equisetum arvense at a concentration of 3%-67% by weight, spergularia rubra at a concentration of 2%-38% by weight, peumus boldus at a concentration of 1.5%-32% by weight, opuntia ficus indica at a concentration of 1%-20% by weight, sideritis angustifolia at a concentration of 1-20% by weight, rosmarinus officinalis at a concentration of 1-20% by weight, cynodon dactylon at a concentration of 1-20% by weight and melissa officinalis at a concentration of 1-20% by weight for use in the treatment of urolithiasis (kidney stones), and prophylaxis of re-formation of stones after passing them, the treatment of gout, and in prevention of excessive amount of salts (urates, oxalates, phosphates, carbonates) in kidneys, the treatment of dysuria phenomenon (pollakiuria, strangury, dysuria, nycturia, low urinary pressure and urinary continence), and of urinary and biliary tract spasm, the treatment of asymptomatic bacteriuria and the treatment of urinary tract infections (cystitis, urethritis, pyelonephritis etc.).. The herbal and pharmaceutical composition of the present invention inhibits stone formation in urolithiasis (kidney stone) patients and eliminates salt crystals that cause stone formation. The herbal and pharmaceutical composition of the present invention inhibits growing of stone in patients having stone smaller than 1 cm in kidney thereby eases the elimination of the stone. Said composition increases urine amount for elimination of the stone and prevents renal colic with its spasmolytic effect. The herbal and pharmaceutical composition of the present invention prevents infection after lithotripsy and eases the elimination of remaining stones. Said composition prevents repeated stone formation after treatment. The herbal and pharmaceutical composition of the present invention eliminates discomfort feeling occurring when patients having urinary tract infection (child and adult) and asymptomatic bacteriuria are urinating, and eliminates inflammation by means of its anti-inflammatory effect. Another purpose of the compositions of the present invention is to overcome the problems encountered in the state of the art by means of the concentration of components and compositions in the formulation.

### Detailed Description of the Invention

The invention is a herbal and pharmaceutical composition for use in the treatment of urolithiasis (kidney stones), and prophylaxis of re-formation of stones after passing them, the treatment of gout, and in prevention of excessive amount of salts (urates, oxalates, phosphates, carbonates) in kidneys, the treatment of dysuria phenomenon (pollakiuria, strangury, dysuria, nycturia, low urinary pressure and urinary continence), and of urinary and biliary tract spasm, the treatment of asymptomatic bacteriuria and the treatment of urinary tract infections (cystitis, urethritis, pyelonephritis etc.), characterized in that said composition comprises Equisetum arvense at a concentration of 3%-67% by weight, spergularia rubra at a concentration of 2%-38% by weight, peumus boldus at a concentration of 1.5%-32% by weight, opuntia ficus indica at a concentration of 1%-20% by weight, sideritis angustifolia at a concentration of 1-20% by weight, rosmarinus officinalis at a concentration of 1-20% by weight, cynodon dactylon at a concentration of 1-20% by weight and melissa officinalis at a concentration of 1-20% by weight.

The composition comprising active substances at the concentrations stated above decreases permeability of capillaries of kidneys, increases vessel width of kidneys and ureters, has an osmotic effect functioning kidney tubules epithelium (do not destabilize ionic equilibrium - potassium protective effect) induces uric acid discharge by inhibiting synthesis and activation of inflammatory mediators thereby inhibiting crystal formation, stone formation and growth and eliminates membranes by normalizing urine pH in order to inhibit leukotriene synthesis and free radical reactions, eliminates bacteria membrane and inhibits bacterial hyaluronidase, decreases urinary protein and inhibits functioning of tubular system, concurrently lifts effectiveness of antibiotic treatment. The inventor has found that optimum performance is provided by the concentration stated during studies conducted.

Another aspect of the composition of the present invention is that it may further comprise a member of the Caryophyllaceae family. In a embodiment of the present invention the member of the Caryophyllaceae family is selected from the group consisting of Herniaria glabra, Herniaria hirsuta and Herniaria fontanessi. In a preferred embodiment of the present invention the member of the Caryophyllaceae family is Herniaria hirsuta. This herb, which can be optionally added, belongs to Caryophyllaceae family. Said herb is known as hairy rupturewort. It grows along dry and stony grounds at altitudes of 1-1700 m. Extracts derived from this herb block formation of calcium oxalate monohydrate crystals and their sticking to kidney epithelial cells. It is used in prophylaxis of formation of calcium oxalate stones from kidney stones. The compositions of the present invention may further comprise Herniaria hirsuta at a concentration of 1% - 20% by weight.

Another factor that can be optionally included in the composition is a member of the Asteraceae family. In a embodiment of the present invention the member of the Asteraceae family is Tridax procumbens. The most typical characteristic of this herb, which is in Asteraceae family is that; it is an antioxidant and by means of this characteristic leading to a notable increase of lipid peroxidation in liver. Moreover, it leads to a reduction in activities of superoxide dismutase, catalase, glutathione peroxidase, glutathione s-transferase and non-enzymatic antioxidants, namely reduced glutathione, enzymic antioxidants such as vitamin C and E. In the compositions of the present invention, when Tridax procumbens is used at the concentration of 50-84% by weight, said composition for the treatment of kidney stones and urinary tract infections is improved.

Another factor that can be optionally included in the composition is a member of the Euphorbiaceae family. In a embodiment of the present invention the member of the Euphorbiaceae family is selected from the group consisting of Phyllanthus sellowianus, Phyllanthus urinaria, Phyllanthus niruni and Phyllanthus corcovadensis. In a preferred embodiment of the present invention the member of the Euphorbiaceae family is Phyllanthus niruri. Phyllanthus niruri has great number of yellow flowers and is a perennial herb and rises 60 cm tall. This herb which is in Euphorbiaceae family has 600 different species in the form of tree, bush and grass. Some of the species have leafy flattened stems and some other have branches with small leaves. It is native to India. It grows in China, Cuba, Nigeria and Guam. Its use for the conventional treatment of diseases such as urinary tract infections and viral infections is popular beside being used frequently for kidney stone diseases. The composition of the present invention may comprise Phyllanthus niruri at a concentration of 1% - 26% by weight.

In the production method of the composition of the present invention primarily raw materials (herbs and excipients) are weighed. Herbs and purified water are subjected to vapor distillation method. By vapor distillation, water is ensured to be vaporized till approximately 135°C by passing through vessel. Water vapor is collected in a vaporizing tower. Then distilled product is collected after passing through a cooler. The composition is a hydrolat obtained by vapor distillation method. After performing addition of excipients homogenization is ensured. Filling and packaging is performed according to desired dosage form. The herbal and pharmaceutical composition of the present invention can be in different forms such as oral solution, syrup, suspension, emulsion, ampule, vial and aerosol. The dosage form of said composition should not be limited to those examples, whereas the product obtained as result of this method should cover all the forms applicable in the art.

In an embodiment of the present invention the herbal and pharmaceutical composition comprises Equisetum arvense at a concentration of 8%-28% by weight, spergularia rubra at a concentration of 5%-16% by weight, peumus boldus at a concentration of 5%-13% by weight, opuntia ficus indica at a concentration of 8%-16% by weight, sideritis angustifolia at a concentration of 8-11% by weight, rosmarinus officinalis at a concentration of 8%-17% by weight, cynodon dactylon at a concentration of 8%-17% by weight and melissa officinalis at a concentration of 8%-17% by weight.

An embodiment of the invention is implemented according to the following formulation concentration:

| Component | Concentration (% by weight) |
|---|---|
| Equisetum arvense | 28 |
| Spergularia rubra | 16 |
| Peumus boldus | 13 |
| Opuntia ficus indica | 8 |
| Sideritis angustifolia | 8 |
| Rosmarinus officinalis | 8 |
| Cynodon dactylon | 8 |
| Melissa officinalis | 8 |
| Preservatives | 0.5 |
| Flavoring | 0.5 |
| Solvent | 2 |
| Total | 100 |

Studies were conducted with a suspension dosage form produced according to the present composition and the above-mentioned production method. The obtained results are indicated below.

Effect of novel herbal and pharmaceutical composition which helps stone elimination in kidney stone disease on Group 1 and Group 2 has been determined by study 1. For Group 1 and Group 2 patient characteristics see Table 1. Elimination duration of kidney stones are indicated in Table 2.

**Table 1: Patients' characteristics**

| **Patient and pathology characteristics** | **Group 1 (n = 31)** | **Group 2 (n = 18)** |
|---|---|---|
| Men | 15 | 7 |
| Women | 16 | 11 |
| Age | 18-65 | 21-64 |
| Calculation measure | >4 mm to <10 mm | >4 mm to <10 mm |
| Stone existence duration | 1-14 days | 1-6 mounts |
| Localization of stone | Ureter | Pelvis, calyx |

**Table 2: Elimination duration of ureter (Group 1) and kidney (Group 2) stones**

| Size (mm) | 3^{rd} Day | | 5^{th} Day | | 10^{th} Day | | 14^{th} Day | | 30^{th} Day | | No elimination | |
|---|---|---|---|---|---|---|---|---|---|---|---|---|
| | 1 | 2 | 1 | 2 | 1 | 2 | 1 | 2 | 1 | 2 | 1 | 2 |
| 4-6 mm | 4 | 0 | 3 | 2 | 4 | 1 | 2 | 0 | 2 | 2 | 0 | 2 |
| 6-8 mm | 3 | 1 | 3 | 1 | 2 | 2 | 2 | 2 | 0 | 2 | 1 | 4 |
| 8-9 mm | 0 | 0 | 1 | 0 | 0 | 0 | 0 | 1 | 0 | 1 | 4 | 5 |

At the result of study, size of the stones seem to be decreased and fragmented in the treatment with the composition of the present invention. Kidney stones are eliminated fragmentarily without urodynamic degradation. The most apparent elimination with (>% 70) 4-7 mm ureteral stones was detected in first 10 days. 8-9 mm urinary stones were eliminated in 20% of the cases.

Representation of symptomatically effectiveness of product and recovery of different urinary disorders are aimed by study 2. Results of the study conducted with 23 patients having dysuria, pollakiuria, nycturia, strangury, urine incontinence disorders are indicated below in Table 3. All of the patients are administered with the composition containing aforementioned concentration. Number of the patients is observed to decrease after 2 months.

**Table 3: Change of number of patients according to use of composition**

| **Symptom** | **Number of patients** | |
|---|---|---|
| | **Beginning** | **2 months later** |
| Dysuria (pain when urinating) | 1 | 0 |
| Pollakiuria (frequent urination) | 5 | 3 |
| Nycturia | 5 | 0 |
| Strangury (difficult urination) | 5 | 4 |
| Urine incontinence | 4 | 3 |
| Total | 23 | 10 |

Another significant point in study 2 showed that in merely two patients having gout arthritis symptoms, disorder representing uricosuric characteristic of preparation that depend on amount of red sandspurry (Spergularia rubra) as well as contributing to content of toxic salts is completely eliminated.

Study 1 and study 2 showed that; clinical effect of the composition is ensured by means of the fact that components of it have active compounds such as volatile oils, flavonoid compounds, phenol carbonic acids and derivatives thereof, phenylpropanoids - rosmarinic acid and ethyl ester, caffeic chlorogenic acid, n-butylamine coumaric, ferulic and sinapic acid; malic acid, alkaloids, glycosides, tannins, pectin, saponins, sterols, coumarin, concentrated vitamins, minerals, trace elements, resin and by their ratios given above.

## Claims

1. Herbal composition comprising Equisetum arvense at a concentration of 3%-67% by weight, Spergularia rubra at a concentration of 2%-38% by weight, Peumus boldus at a concentration of 1.5%-32% by weight, Opuntia ficus indica at a concentration of 1%-20% by weight, Sideritis angustifolia at a concentraiton of 1-20% by weight, Rosmarinus officinalis at a concentration of 1-20% by weight, Cynodon dactylon at a concentration of 1-20% by weight and Melissa officinalis at a concentration of 1-20% by weight for use in the treatment of urolithiasis (kidney stones), and prophylaxis of re-formation of stones after passing them, for the treatment of gout, and for prevention of excessive amount of salts (urates, oxalates, phosphates, carbonates) in kidneys, for the treatment of dysuria phenomenon (pollakiuria, strangury, dysuria, nycturia, low urinary pressure and urinary incontinence), and of urinary and biliary tract spasm, for the treatment of asymptomatic bacteriuria and for the treatment of urinary tract infections (cystitis, urethritis, pyelonephritis).

2. Herbal composition for use according to claim 1, **characterized in that** said composition comprises Equisetum arvense at a concentration of 8%-28% by weight, spergularia rubra at a concentration of 5%-16% by weight, peumus boldus at a concentration of 5%-13% by weight, opuntia ficus indica at a concentration of 8%-16% by weight, sideritis angustifolia at a concentration of 8-11% by weight, rosmarinus officinalis at a concentration of 8%-17% by weight, cynodon dactylon at a concentration of 8%-17% by weight and melissa officinalis at a concentration of 8%-17% by weight.

3. Herbal composition for use according to claims 1 or 2, **characterized in that** it further comprises a member of the Caryophyllaceae family.

4. Herbal composition for use according to claim 3, **characterized in that** the member of the Caryophyllaceae family is Herniaria hirsuta at a concentration of 1%-20% by weight.

5. Herbal composition for use according to any one of claims 1 to 4, **characterized in that** it further comprises a member of the Asteraceae family.

6. Herbal composition for use according to claim 5, **characterized in that** the member of the Asteraceae family is Tridax procumbens at a concentration of 50%-84% by weight.

7. Herbal composition for use according to any one of claims 1 to 6, **characterized in that** it further comprises a member of the Euphorbiaceae family.

8. Herbal composition for use according to claim 7, **characterized in that** the member of the Euphorbiaceae family is Phyllanthus niruri at a concentration of 1%-26% by weight.

9. Herbal composition for use according to any one of claims 1 to 8, **characterized in that** it has a dosage form selected from the group consisting of oral solution, syrup, suspension, emulsion, ampul and aerosol.

10. Method for production of an herbal composition for use according to any one of claims 1 to 9, **characterized in that** it comprises the steps of weighing herbs and excipients, subjecting herbs and water to vapor distillation, vaporizing purified water at 135°C, collecting water vapor in vapor tower, obtaining hydrolat, addition of excipients and homogenization.

## Patentansprüche

1. Kräuterzusammensetzung, umfassend Equisetum arvense in einer Konzentration von 3-67 Gew.-%, Spergularia rubra in einer Konzentration von 2-38 Gew.-%, Peumus boldus in einer Konzentration von 1,5-32 Gew.-%, Opuntia ficus indica in einer Konzentration von 1-20 Gew.-%, Sideritis angustifolia in einer Konzentration von 1-20 Gew.-%, Rosmarinus officinalis in einer Konzentration von 1-20 Gew.-%, Cynodon dactylon in einer Konzentration von 1-20 Gew.-% und Melissa officinalis in einer Konzentration von 1-20 Gew.-% zur Verwendung bei der Behandlung von Urolithiasis (Nierensteinen) und zur Prophylaxe der Neubildung von Steinen nach dem Passieren derselben, zur Behandlung von Gicht und zur Vorbeugung einer übermäßigen Menge an Salzen (Uraten, Oxalaten, Phosphaten, Carbonaten) in den Nieren, zur Behandlung des Phänomens der Dysurie (Pollakisurie, Strangurie, Dysurie, Nykturie, niedrigem Harndruck und Harninkontinenz) und des Spasmus der Harn- und Gallenwege, zur Behandlung der asymptomatischen Bakteriurie und zur Behandlung von Harnwegsinfektionen (Zystitis, Urethritis, Pyelonephritis).

2. Kräuterzusammensetzung zur Verwendung nach Anspruch 1, **dadurch gekennzeichnet, dass** die Zusammensetzung Equisetum arvense in einer Konzentration von 8-28 Gew.-%, Spergularia rubra in einer Konzentration von 5-16 Gew.-%, Peumus boldus in einer Konzentration von 5-13 Gew.-%, Opuntia ficus indica in einer Konzentration von 8-16 Gew.-%, Sideritis angustifolia in einer Konzentration von 8-11 Gew.-%, Rosmarinus officinalis in einer Konzentration von 8-17 Gew.-%, Cynodon dactylon in einer Konzentration von 8-17 Gew.-% und Melissa officinalis in einer Konzentration von 8-17 Gew.-% umfasst.

3. Kräuterzusammensetzung zur Verwendung nach Anspruch 1 oder 2, **dadurch gekennzeichnet, dass** sie ferner ein Mitglied der Familie der Caryophyllaceae umfasst.

4. Kräuterzusammensetzung zur Verwendung nach Anspruch 3, **dadurch gekennzeichnet, dass** das Mitglied der Familie der Caryophyllaceae Herniaria hirsuta in einer Konzentration von 1-20 Gew.-% ist.

5. Kräuterzusammensetzung zur Verwendung nach einem der Ansprüche 1 bis 4, **dadurch gekennzeichnet, dass** sie ferner ein Mitglied der Familie der Asteraceae umfasst.

6. Kräuterzusammensetzung zur Verwendung nach Anspruch 5, **dadurch gekennzeichnet, dass** das Mitglied der Familie der Asteraceae Tridax procumbens in einer Konzentration von 50-84 Gew.-% ist.

7. Kräuterzusammensetzung zur Verwendung nach einem der Ansprüche 1 bis 6, **dadurch gekennzeichnet, dass** sie ferner ein Mitglied der Familie der Euphorbiaceae umfasst.

8. Kräuterzusammensetzung zur Verwendung nach Anspruch 7, **dadurch gekennzeichnet, dass** das Mitglied der Familie der Euphorbiaceae Phyllanthus niruri in einer Konzentration von 1-26 Gew.-% ist.

9. Kräuterzusammensetzung zur Verwendung nach einem der Ansprüche 1 bis 8, **dadurch gekennzeichnet, dass** sie eine Darreichungsform aufweist, ausgewählt aus der Gruppe bestehend aus oraler Lösung, einem Sirup, einer Suspension, einer Emulsion, einer Ampulle und einem Aerosol.

10. Verfahren zur Herstellung einer Kräuterzusammensetzung zur Verwendung nach einem der Ansprüche 1 bis 9, **dadurch gekennzeichnet, dass** es die Schritte des Wiegens von Kräutern und Hilfsstoffen, des Unterziehens von Kräutern und Wasser einer Dampfdestillation, des Verdampfens von gereinigtem Wasser bei 135 °C, des Auffangens des Wasserdampfes in einem Dampfturm, des Erhaltens von Hydrolat, der Zugabe von Hilfsstoffen und des Homogenisierens umfasst.

## Revendications

1. Composition à base d'herbes médicinales comprenant Equisetum arvense à une concentration de 3% à 67% en poids, Spergularia rubra à une concentration de 2% à 38% en poids, Peumus boldus à une concentration de 1,5% à 32% en poids, Opuntia ficus indica à une concentration de 1 à 20% en poids, Sideritis angustifolia à une concentration de 1 à 20% en poids, Rosmarinus officinalis à une concentration de 1 à 20% en poids, Cynodon dactylon à une concentration de 1 à 20% en poids et Melissa officinalis à une concentration de 1à 20% en poids pour le traitement de l'urolithiase (calculs rénaux) et la prophylaxie de la reformation des calculs après leur élimination, pour le traitement de la goutte et pour la prévention des excès de sels (urates, oxalates, phosphates, carbonates) dans les reins, pour le traitement des phénomènes de dysurie (pollakiurie, strangurie, dysurie, nycturie, hypotension et incontinence urinaire) et des spasmes des voies urinaires et biliaires, pour le traitement de la bactériurie asymptomatique et pour le traitement des infections des voies urinaires (cystite, urétrite, pyélonéphrite).

2. Composition à base d'herbes médicinales pour utilisation selon la revendication 1, **caractérisée en ce que** ladite composition comprend Equisetum arvense à une concentration de 8% à 28% en poids, spergularia rubra à une concentration de 5% à 16% en poids, peumus boldus à une concentration de 5% à 13% en poids, opuntia ficus indica à une concentration de 8% à 16% en poids, sideritis angustifolia à une concentration de 8 à 11% en poids, rosmarinus officinalis à une concentration de 8% à 17% en poids, cynodon dactylon à une concentration de 8% à 17% en poids et melissa officinalis à une concentration de 8% à 17% en poids.

3. Composition à base d'herbes médicinales pour utilisation selon les revendications 1 ou 2, **caractérisée en ce qu'**elle comprend en outre un membre de la famille des Caryophyllacées.

4. Composition à base d'herbes médicinales pour utilisation selon la revendication 3, **caractérisée en ce que** le membre de la famille des Caryophyllaceae est Herniaria hirsuta à une concentration de 1 % à 20 % en poids.

5. Composition à base d'herbes médicinales pour utilisation selon l'une quelconque des revendications 1 à 4, **caractérisée en ce qu'**elle comprend en outre un membre de la famille des Astéracées.

6. Composition à base d'herbes médicinales pour utilisation selon la revendication 5, **caractérisée en ce que** le membre de la famille des Asteraceae est Tridax procumbens à une concentration de 50% à 84% en poids.

7. Composition à base d'herbes médicinales pour utilisation selon l'une quelconque des revendications 1 à 6, **caractérisée en ce qu'**elle comprend en outre un membre de la famille des Euphorbiaceae.

8. Composition à base d'herbes médicinales pour utilisation selon la revendication 7, **caractérisée en ce que** le membre de la famille des Euphorbiaceae est Phyllanthus niruri à une concentration de 1 % à 26 % en poids.

9. Composition à base d'herbes médicinales pour utilisation selon l'une quelconque des revendications 1 à 8, **caractérisée par** une forme de dosage choisie dans le groupe constitué des solutions orales, les sirops, les suspensions, les émulsions, les ampoules et les aérosols.

10. Procédé de production d'une composition à base d'herbes médicinales pour utilisation selon l'une quelconque des revendications 1 à 9, **caractérisé en ce qu'**il comprend les étapes consistant à : peser des plantes et des excipients, soumettre les plantes et l'eau à une distillation à la vapeur, vaporiser l'eau purifiée à 135°C, recueillir la vapeur d'eau dans une tour à vapeur, obtenir l'hydrolat, ajouter les excipients et homogénéiser.
